# EUROPEAN PATENT SPECIFICATION

(11) **EP 2 300 541 B1**
(45) Date of publication and mention of the grant of the patent: **12.02.2014**
(21) Application number: 09769124.0
(22) Date of filing: 10.06.2009
(51) Int. Cl.: C09C 1/46, A61Q 19/00, A61K 8/02, A61K 8/19, B82Y 30/00, C08K 3/04, C09D 17/00, C09D 7/12, C09C 1/00

(54) **PIGMENT MIXTURES**
PIGMENTMISCHUNGEN
MÉLANGES DE PIGMENTS

(30) Priority: 24.06.2008 EP 08158889
(43) Date of publication of application: 30.03.2011
(73) Proprietor: BASF SE, 67056 Ludwigshafen (DE)
(72) Inventor: HALL-GOULLE, Véronique, CH-4143 Dornach (CH); ZILLHARDT, Rebekka, 79599 Wittlingen (DE); BUGNON, Philippe, CH-1724 Le Mouret (CH)
(86) International application number: PCT/EP2009/057130
(87) International publication number: WO 2009/156275

(56) References cited:
- EP-A- 0 439 107
- WO-A-03/006558
- US-A1- 2004 225 032

## Description

The present invention relates to novel pigment mixtures, comprising two different components A and B, wherein component A is a graphite in the form of platelets, which have an average particle size of below 50 µm (microns) and a thickness below 90 nm, and component B is an organic, or inorganic pigment.

Graphite nanostructures in the form of platelets are known. Reference is made, for example, to WO03024602, which describes graphite nanostructures in the form of platelets, wherein a majority of said platelets have an aspect ratio of at least 1,500 : 1. The majority of the platelets have a specific surface area of at least about 5 m²/g and an average thickness of less than 100 nm. The graphite nanostructures are obtained by a method for fracturing graphite particles into platelets, comprising: introducing the graphite into a high-pressure flaking mill, wherein said high-pressure flaking mill causes a hydro-wedging effect that overcomes the Van der Waals forces of the particles and fractures said particles into platelets. The resulting graphite nanostructures can be added to conventional polymers to create polymer composites having increased mechanical characteristics, including an increased flexural modulus, heat deflection temperature, tensile strength, electrical conductivity, and notched impact strength.

US4477608 discloses a composition, which can be processed into moulded products, which comprises (a) a thermoplastic high-molecular organic material selected from the group consisting of polyvinyl chloride, polyethylene, polypropylene, polystyrene, polycarbonate, polyacrylate, linear polyester, polyether, linear polyurethane and copolymers thereof, (b) 0.001 to 3.0% by weight, relative to the high-molecular organic material, of graphite of a particle diameter of less than 100 µm (microns), and (c) 0.001 to 10.0% by weight, relative to the high-molecular organic material, of one or more organic pigments; polymer-soluble dyes; or inorganic pigments selected from the group consisting of iron oxide, antimony yellow, lead chromates, molybdenum red, ultramarine blue, cobalt blue, manganese blue, chromium oxide green, hydrated chrome oxide green, cobalt green, cadmium sulfide, zinc sulfide, arsenic disulfide, mercury sulfide, antimony trisulfide and cadmium sulfoselenides.

A graphite which is in flake- or lamella-form with a diameter of up to 20 µm and a thickness of up to 4 µm is preferably used according to US4477608.

EP0439107 relates to a pigment blend for use in a coating composition comprising an essentially transparent pigment or a pearlescent pigment in combination with an effective amount of a laminar graphite to cause a hue shift in the coating composition without substantially reducing the chromaticity. Laminar graphite as defined in EP0439107 refers to a flake shaped black pigment, having an average particle size of about 3.5 µm (microns), which is commercially available as Graphitan® sold by Ciba. The thickness of the Graphitan particles is in the range of from 200 to 400 nm.

US2004225032A1 relates to an erasable ink for use in a writing instrument, comprising: a solvent, a shear-thinning additive, and a graphite flake pigment having an average thickness of less than about 0.25 µm (microns) dispersed in the solvent, wherein said ink has a shear-thinning index of between about 0.01 and 0.8. The ink is said to be substantially free of colorants other than said graphite flake pigment. Preferably, the graphite particles are flake natural graphite. Examples of suitable graphite particles include, but are not limited to, those sold under the trade names, Micro750 and Micro790 (flake), Micro150 and Micro190 (amorphous), Micro250 and Micro290 (primary synthetic), and Micro450 and Micro490 (secondary synthetic), available from Graphite Mills, Inc. (Asbury Graphite Mills, N.J.). In Table 1 of US2004/0225032 mixtures of Dichrona® BG (mica coated with TiO₂ and iron blue) and Graphite M790 are described.

US6267810 relates to a pigment mixture comprising a component A and a component B, wherein component A comprises Al₂O₃ flakes coated with one or more metals, metal oxides or metal sulfides, and wherein component B comprises special-effect pigments.

Component B can comprise i) one or more of metal platelets coated with one or more metal oxides, ii) graphite platelets, iii) aluminum platelets, iv) phyllosilicates, v) Fe₂O₃-flakes, SiO₂-flakes, or TiO₂ -flakes uncoated or coated with one or more metal oxides, vi) glass platelets and or vii) ceramic platelets.

US6632275 relates to a pigment mixture comprising two different components A and B mixed in a weight ratio of A:B of from 1:10 to 10:1, wherein component A is SiO₂ flakes coated with one or more metal oxides and/or metals and component B is a special effect pigment comprising one or more of metal platelets optionally coated with one or more metal oxides, graphite platelets, optionally coated aluminum platelets, optionally coated Al₂O₃ flakes, Fe₂O₃ flakes, TiO₂ flakes, BiOCl, glass platelets and ceramic platelets.

US6773499 relates to a composition comprising a binder and a pigment mixture, wherein the pigment mixture comprises two different components A and B mixed in a weight ratio of A:B of from 1:10 to 10:1, wherein component A is SiO₂ flakes coated with one or more metal oxides and/or metals and component B is a special effect pigment comprising one or more of metal platelets optionally coated with one or more metal oxides, graphite platelets, optionally coated aluminum platelets, optionally coated phyllosilicates, optionally coated Al₂O₃ flakes, Fe₂O₃ flakes, TiO₂ flakes, BiOCl, glass platelets and ceramic platelets, and wherein the composition is substantially solvent-free and in the form of free-flowing granules.

According to G. Pfaff et al. "Special Effect Pigments" 1998, p. 47 some grey shades can be produced by mixing silver white pearl pigments with carbon black. As such mixtures are difficult to process. Merck favours carbon inclusion pigments, i.e. mica pigments having dispersed in the TiO₂ layer the carbon black particles (US-A-4,076,551 etc.).

Except for metal-pigment colorations, most of effect pigment metallic-like colorations are transparent. While the addition of traditional black pigments (carbon black) or black dyes does bring effectively the needed opacity, it kills completely the effect. The addition of traditional platelet-like graphite (laminar graphite), such as, for example, commercial "GRAPHITAN" does not bring the opacity at a reasonable concentration, and is useless at higher concentration due to bad rheological behaviour.

The use of graphite nanoplatelets (graphene, component A) in a pigment mixture with organic and/or inorganic pigments, especially effect pigments (component B), allows the preparation of metallic like colorations with a maximal opacity (background substrate disappears totally) while keeping a good rheological behaviour (low concentration use).

Accordingly, the present invention relates to pigment mixtures comprising two different components A and B, wherein
component A is a graphite in the form of platelets (graphite nanoplatelets), which have an average particle size of below 50 µm (microns) and a thickness below 90 nm, and component B is an organic, or inorganic pigment and their use in varnishes, paints, printing inks, masterbatches, plastics and cosmetics formulations.

The graphite in the form of platelets (graphite nanoplatelets) has a high covering power which adds opacity to the layer/matrix into which it is included and preserves the effect of the effect pigment.

The present invention relates to pigment mixtures consisting of at least two components, component A and B.

Component A being an exfoliated expanded graphite (graphite oxide) and component B being organic pigment or inorganic pigment, especially effect pigment.

Component A is a graphite in the form of platelets (graphite nanoplatelets), which have an average particle size of below 50 µm (microns) and a thickness below 90 nm, especially below 90 nm. Graphite nanoplatelets and the preparation thereof are, for example, described in PCT/EP2009/052127, WO2003024602 and US2007092432.

Preferably, greater than 95% of the graphite nanoplatelets have a thickness below 50 nm.

Preferably, greater than 95% of the graphite nanoplatelets have a thickness below 20 nm.

Graphite nanoplatelets, which can advantageously be used in the pigment mixtures of the present invention, are described in PCT/EP2009/052127.
The graphite nanoplatelets are produced by a process which comprises thermal plasma expansion of intercalated graphite to produce expanded graphite followed by exfoliation of the expanded graphite, where the exfoliation step is selected from ultrasonication, wet milling and controlled caviation. Greater than 95% of the graphite nanoplatelets obtained by the process have a thickness of from about 0.34 nm to about 50 nm and a length and width of from about 500 nm to about 50 µm (microns).

Intercalated graphite is disclosed for example in US4,895,713.

The intercalated graphite is also referred to as expandable graphite flakes or intumescent flake graphite. It is commercially available as GRAFGUARD from GrafTech International Ltd, Parma, Ohio. Expandable graphite is also available from Asbury Carbons, Asbury, New Jersey. Suitable grades are GRAFGUARD 220-80N, GRAFGUARD 160-50N, ASBURY 1721 and ASBURY 3538. These products are prepared by intercalating natural graphite with a mixture of sulfuric and nitric acids.

Plasma reactors are known and disclosed for instance in US5,200,595. The present invention employs an RF (radio frequency) induction plasma torch. Induction plasma torches are available for instance from Tekna Plasma Systems Inc., Sherbrooke, Quebec.

An advantage of the plasma expansion process is that it is a continuous, high throughput process. It is more efficient compared to an electric/gas furnace or microwave oven.

The graphite nanoplatelets prepared according to the process described in PCT/EP2009/052127 are such that greater than 95% have a thickness of from about 0.34 nm to about 50 nm and a length and width of from about 500 nm to about 50 µm (microns). For instance, greater than 90% have a thickness of from about 3 nm to about 20 nm and a length and width of from about 1 µm (micron) to about 5 µm (microns). For instance, greater than 90% have a thickness of from about 3 nm to about 20 nm and a length and width of from about 1 to about 30 µm (microns). For instance, greater than 90% have a thickness of from about 0.34 nm to about 20 nm and a length and width of from about 1 to about 30 µm (microns). The aspect ratio is at least 50 and may be as high as 50,000. That is 95% of the particles have this aspect ratio. For instance, the aspect ratio of 95% of the particles is from about 500 to about 10,000, for instance from about 600 to about 8000, or from about 800 to about 6000.

It is understood that component B is different than component A. Component B is an organic pigment, or an inorganic pigment, especially an effect pigment.

(Multi)layered structures leading to interference colors (effect pigments) are often referred to as special-effect pigments, luster or nacreous pigments and well known in the art and commercially available under such tradenames as Xymara® available from Ciba Chemicals Inc.

In principle component B might comprise all platelet-like effect pigments, such as, for example, platelet-like iron oxide, bismuth oxychloride or platelet-like materials coated with colored or colorless metal oxides, such as, for example, natural or synthetic micas, other laminated silicates such as talc, kaolin or sericite or glass platelets can be used. Mica flakes coated with metal oxides such as are disclosed, for example, in US-A-3,087,828 and 3,087,829 are particularly preferred as substrates. Metal oxides are both colorless, highly refractive metal oxides, such as, in particular, titanium dioxide and/or zirconium dioxide, as well as colored metal oxides, such as, for example, chromium oxide, nickel oxide, copper oxide, cobalt oxide and in particular iron oxides, such as, for example, Fe₂O₃, or Fe₃O₄, or mixtures of such metal oxides. Such metal oxide/mica pigments are commercially available under the tradenames Afflair® and Iriodin®. According to EP-A-373575 these substrates are coated with an optionally hydrated silica layer or with a layer of another insoluble silicate such as, for example, aluminum silicate.

These (multilayer) structures frequently are formed from a core of natural micaceous iron oxide (for example as in WO99/48634), synthetic and doped micaceous iron oxide (for example as in EP-A-068311), mica (muscovite, phlogopite, fluorophlogopite, synthetic fluorophlogopite, talc, kaolin), basic lead carbonate, flaky barium sulfate, SiO₂, Al₂O₃, TiO₂, glass, ZnO, ZrO₂, SnO₂, BiOCl, chromium oxide, BN, MgO flakes, Si₃N₄, graphite, pearlescent pigments (including those which react under the fluidized bed conditions to nitrides, oxynitrides or by reduction to suboxides etc.) (for example EP-A-0948571, U.S. Patent Nos. 6,773,499, 6,508,876, 5,702,519, 5,858,078, WO98/53012, WO97/43348, US-B-6,165,260, DE-A-1519116, WO97/46624, EP-A-0509352), pearlescent (multilayer) pigments (for example EP-A-0948572, EP-A-0882099, US-A-5,958,125, 6,139,613), coated or uncoated SiO₂ spheres (for example known from EP-A-0803550, EP-A-1063265, JP-A-11322324), EP-A-0803550, EP-A-1063265, JP-A-11322324). Particularly preferred cores are mica, SiO₂ flakes, Al₂O₃ flakes, TiO₂ flakes, Fe₂O₃ flakes, BiOCl and glass flakes.

The glass flake cores for the purpose of the invention include any of the known grades such as A-glass, E-glass (high resistivity makes E-glass suitable for electrical laminates), C-glass and ECR-glass (corrosion grade glass) materials.

For example, component B particle may be a platelet-like (multilayered) structure such as:

| | | | |
|---|---|---|---|
| TRASUB | TiO₂ | | |
| TRASUB | TiO₂ | Fe₂O₃ | |
| TRASUB | TiO₂ | Fe₃O₄ | |
| TRASUB | titanium suboxide(s) | | |
| TRASUB | TiO₂ | TiN | |
| TRASUB | TiO₂ | SiO₂ | |
| TRASUB | TiO₂ | titanium suboxide(s) | |
| TRASUB | TiO₂ | TiON | TiN |
| TRASUB | TiO₂ | SiO₂ | TiO₂ |
| TRASUB | TiO₂ | SiO₂ | silicon suboxide(s) |
| TRASUB | TiO₂ | SiO₂ | Fe₂O₃ |
| TRASUB | TiO₂ | SiO₂ | TiO₂/Fe₂O₃ |
| TRASUB | TiO₂ | SiO₂ | (Sn,Sb)O₂ |
| TRASUB | SnO₂ | TiO₂ | |
| TRASUB | SnO₂ | TiO₂ | Fe₂O₃ |
| TRASUB | (Sn,Sb)O₂ | SiO₂ | TiO₂ |
| TRASUB | Fe₂O₃ | SiO₂ | (Sn,Sb)O₂ |
| TRASUB | TiO₂/Fe₂O₃ | SiO₂ | TiO₂/Fe₂O₃ |
| TRASUB | Cr₂O₃ | SiO₂ | TiO₂ |
| TRASUB | Fe₂O₃ | | |
| TRASUB | Fe₂O₃ | SiO₂ | TiO₂ |
| TRASUB | titanium suboxide(s) | SiO₂ | titanium suboxide(s) |
| TRASUB | TiO₂ | SiO₂ | TiO₂ + SiO₂ + TiO₂ |
| TRASUB | TiO₂ + SiO₂ + TiO₂ | SiO₂ | TiO₂ + SiO₂ + TiO₂ |
| TRASUB | TiO₂ | Al₂O₃ | TiO₂ |
| TRASUB | Fe₂TiO₅ | SiO₂ | TiO₂ |
| TRASUB | TiO₂ | SiO₂ | Fe₂TiO₅/ TiO₂ |
| TRASUB | TiO₂ | SiO₂ | MoS₂ |
| TRASUB | TiO₂ | SiO₂ | Cr₂O₃ |
| TRASUB | TiO₂ | SiO₂ | TiO₂ + SiO₂ + TiO₂ + Prussian Blue |
| TRASUB | TiO₂ | STL | |

wherein TRASUB is a semitransparent, or transparent substrate having a low index of refraction selected from the group consisting of natural, or synthetic mica, another layered silicate, glass, Al₂O₃, SiO₂, SiO₂, SiO₂/SiOₓ/SiO₂ (0.03 ≤ x ≤ 0.95), SiO_{1.40-2.0}/SiO_{0.700.99}/SiO_{1.40-2.0}, or Si/SiO_{z} with 0.70 ≤ z ≤ 2.0, and
STL is a semi-transparent layer selected from the group consisting of a semi-transparent metal layer of Cu, Ag, Cr, or Sn, or a semi-transparent silicon suboxide(s), titanium suboxide(s) or carbon layer.

The (multilayered) pigments above may also include an absorption pigment as an additional layer. For example a further coating with Prussian blue or red-carmine on an interference pigment allows for striking color effects.

Pigments based on TiO₂ and/or Fe₂O₃ coated, platelet-like, transparent substrates are preferred, wherein those are most preferred, wherein the thickness of the TiO₂ and/or Fe₂O₃ layer results in a silver-like color, a gold-like color, a bronze-like color, a violet-like color, a blue-like color and a green-like color.

Other layered structures envisioned for component B are:
(a) a transparent substrate having a low index of refraction selected from the group consisting of natural, or synthetic mica, another layered silicate, glass, Al₂O₃, SiO_{z}, SiO₂, SiO₂/SiOₓ/SiO₂ (0.03 ≤ x ≤ 0.95), SiO_{1.40-2.0}/SiO_{0.70-0.99}/SiO_{1.40-2.0}, or Si/SiO_{z} with 0.70 ≤ z ≤ 2.0 and
(b) a layer of a metal oxide of high refractive index on the substrate selected from the group consisting of ZrO₂, Fe₂O₃, or TiO₂; or
(a) a transparent substrate having a low index of refraction selected from the group consisting of natural, or synthetic mica, another layered silicate, glass, Al₂O₃, SiO_{z}, especially SiO₂, SiO₂/SiOₓ/SiO₂ (0.03 ≤ x ≤ 0.95), SiO_{1.40-2.0}/SiO_{0.70-0.99}/SiO_{1.40-2.0}, or Si/SiO_{z} with 0.70 ≤ z ≤ 2.0, and
(b) a reflecting layer or a semitransparent layer, or a semitransparent metal layer; or
(a) a platelet shaped titanium dioxide substrate,
(b) a layer of Fe₂O₃, Fe₃O₄, FeOOH, Cr₂O₃, CuO, Ce₂O₃, Al₂O₃, SiO₂, BiVO₄, NiTiO₃, CoTiO₃ and also antimony-doped, fluorine-doped or indium-doped tin oxide; or
(a) a platelet shaped iron oxide substrate,
b) a colorless coating having a refractive index n ≤ 1.8, and
c) a colorless coating having a refractive index n ≥ 2.0.

Alternative (multilayer) structures for component B might comprise flakes comprising layers (a), (b) and optionally (c):
(a) a metallic platelet-shaped substrate selected from the group consisting of titanium, silver, aluminum, copper, chromium, iron, germanium, molybdenum, tantalum, or nickel, and
(b) a layer of a metal oxide of low refractive index or of high refractive index on the substrate
(c) an optional layer comprising a semi-transparent metal oxide selected from the group consisting of SiO_{z}, SiO₂/SiO_{z}, titanium suboxide(s), TiO₂/titanium suboxide(s) and 0.70 ≤ z ≤ 2.0.

Moreover, flakes comprising layered structures of (a), (b) and (c) below are preferred:
(a) a transparent substrate having a low index of refraction selected from the group consisting of natural, or synthetic mica, another layered silicate, glass, Al₂O₃, SiO_{z}, SiO₂, SiO₂/SiOₓ/Si0₂ (0.03≤ x ≤ 0.95), SiO_{1.40-2.0}/SiO_{0.70-0.99}/SiO_{1.40-2.0}, or Si/SiO_{z} with 0.70 ≤ z ≤ 2.0, and
(b) a titanium dioxide layer,
(c) a layer of hydrous aluminum oxide, a layer of hydrated zirconium oxide, a top layer comprising hydrated zirconium oxide obtained by hydrolysis in the presence of a hypophosphite, and a hydrated metal oxide, or a combination of hydrated cerium and aluminum oxides, or a layer (topcoat) which contains a polysiloxane and a rare earth metal compound.

Furthermore platelet-like particles, comprising
(a) a core and
(b) a polymeric coating, comprising nitrogen and carbon atoms, on the surface of the flakes are envisioned.

Additionally, platelet-like particles, comprising
(a) a substrate, and
(b) a layer of a metal nitride/oxy nitride, titanium suboxide(s), SiO_{z} or SiO₂/SiO_{z}, wherein 0.70 ≤ z ≤ 2.0, are possible as component B.

The (multilayered) structures may be spherical, rod-like or platelet-shaped substrates. Platelet, flakey shapes are preferred.

The component B may also be an organic color pigment or a conventional inorganic pigment.

Suitable colored pigments especially include organic pigments selected from the group consisting of azo, azomethine, methine, anthraquinone, phthalocyanine, perinone, perylene, diketopyrrolopyrrole, thioindigo, dioxazine iminoisoindoline, dioxazine, iminoisoindolinone, quinacridone, flavanthrone, indanthrone, anthrapyrimidine and quinophthalone pigments, or a mixture or solid solution thereof; especially a dioxazine, diketopyrrolopyrrole, quinacridone, phthalocyanine, indanthrone or iminoisoindolinone pigment, or a mixture or solid solution thereof.

Colored organic pigments of particular interest include C.I. Pigment Red 202, C.I. Pigment Red 122, C.I. Pigment Red 179, C.I. Pigment Red 170, C.I. Pigment Red 144, C.I. Pigment Red 177, C.I. Pigment Red 254, C.I. Pigment Red 255, C.I. Pigment Red 264, C.I. Pigment Brown 23, C.I. Pigment Yellow 109, C.I. Pigment Yellow 110, C.I. Pigment Yellow 147, C.I. Pigment Orange 61, C.I. Pigment Orange 71, C.I. Pigment Orange 73, C.I. Pigment Orange 48, C.I. Pigment Orange 49, C.I. Pigment Blue 15, C.I. Pigment Blue 60, C.I. Pigment Violet 23, C.I. Pigment Violet 37, C.I. Pigment Violet 19, C.I. Pigment Green 7, C.I. Pigment Green 36, the 2,9-dichloro-quinacridone in platelet form described in WO08/055807, or a mixture or solid solution thereof.

Plateletlike organic pigments, such as plateletlike quinacridones, phthalocyanine, fluororubine, dioxazines, red perylenes or diketopyrrolopyrroles can advantageously be used as component B.

Suitable colored pigments also include conventional inorganic pigments; especially those selected from the group consisting of metal oxides, antimony yellow, lead chromate, lead chromate sulfate, lead molybdate, ultramarine blue, cobalt blue, manganese blue, chrome oxide green, hydrated chrome oxide green, cobalt green and metal sulfides, such as cerium or cadmium sulfide, cadmium sulfoselenides, zinc ferrite, bismuth vanadate, Prussian blue, Fe₃O₄, carbon black and mixed metal oxides. Examples of commercially available inorganic pigments are BAYFERROX® 3920, BAYFERROX® 920, BAYFERROX® 645T, BAYFERROX® 303T, BAYFERROX® 110, BAYFERROX® 110 M, CHROMOXIDGRUEN GN, and CHROMOXIDGRUEN GN-M.

It is preferred that component B particle is plate-like or alternatively described as flakes or parallel structures. Generally the flakes have a length of from 1 µm to 5 mm, a width of from 1 µm to 5 mm, and a thickness of from 20 nm to 2 µm, and a ratio of length to thickness of at least 2 : 1, the particles having two substantially parallel faces, the distance between which is the shortest axis of the core.

The component B flakes of the present invention are not of a uniform shape. Nevertheless, for purposes of brevity, the flakes will be referred to as having a "diameter". The flakes have a thickness of from 20 to 2000 nm, especially from 50 to 1000 nm. It is presently preferred that the diameter of the flakes be in a preferred range of about 1-60 µm with a more preferred range of about 5-40 µm.

Preferred component B particles are any high aspect ratio materials, such as platelets (flakes), rod-like materials and fibers. The aspect ratio is at least 10 to 1. The term "aspect ratio" refers to the ratio of the maximum (length) to the minimum dimension (thickness) of a particle.

Thus, the aspect ratio of the flakes of the present invention is in a preferred range of about 2.5 to 625.

The weight ratio of component A to component B may be any ratio. For example the ratio may be about 1:1 to about 1:200. Preferably, the ratio is about 1:10 to about 1:200, more preferably about 1:10 to about 1:30.

The two components A and B may be mixed to form a pigment composition (physical mixture).

Alternatively, component(s) A may be coated or deposited onto component B.

A pigment mixture (composite pigment) may also be prepared by a process comprising spray-drying an aqueous suspension consisting of discrete particles of component A and B (cf. US5562763).

The pigment mixtures of the invention may be incorporated in coatings, ceramics, glasses plastics, films, agricultural films, button pastes, masterbatches, seed coatings, printing inks, cosmetics and personal care products. Accordingly, the present invention relates to coatings, varnishes, plastics, paints, printing inks, masterbatches, ceramics or glasses, cosmetics or personal care products, comprising the particles of the present invention, or the pigment mixture of the present invention.

The concentration of the pigment mixture in the system in which it is to be used forpigmenting is generally between 0.01 and 75% by weight, preferably between 0.1 and 60% by weight, based on the overall solids content of the system. This concentration is generally dependent on the specific application.

Plastics comprising the pigment mixture of the invention in amounts of from 0.1 to 50% by weight, in particular from 0.5 to 7% by weight, are frequently notable for a bluish/grey or bluish/black lustrous metallic effect.

In the coating sector, especially in automotive finishing, the pigment mixture is employed in amounts of 0.5-10% by weight. The proportion in which the component A are mixed with component B, depends on the desired effect.

The invention likewise provides pigment preparations comprising components A and B with binders and, if desired, additives, the said preparations being in the form of substantially solvent-free, free-flowing granules. Such granules contain up to 95% by weight of the pigment mixture. A pigment preparation in which the pigment mixture of the invention is pasted up with a binder and with water or an organic solvent, with or without additives, and the paste is subsequently dried and brought into a compact particulate form, e.g. granules, pellets, briquettes, a masterbatch or tablets.

The mixtures are highly suitable for coloring plastics or high molecular weight materials which can be further processed to fibers, cast and molded articles, films or coating compositions such as solvent or water based coatings, which are for example conventionally employed in the automobile industry.

Thus, the high molecular weight organic material may be an industrial paint, automotive paint, molded article or film.

Suitable high molecular weight organic materials include thermoplastics, thermoset plastics or elastomers, natural resins or casein for example, cellulose ethers; cellulose esters such as ethyl cellulose; linear or crosslinked polyurethanes; linear, crosslinked or unsaturated polyesters; polycarbonates; polyolefins such as polyethylene, polypropylene, polybutylene or poly-4-methylpent-1-ene; polystyrene; polysulfones; polyamides; polycycloamides; polyimides; polyethers; polyether ketones such as polyphenylene oxides; and also poly-p-xylene; polyvinyl halides such as polyvinyl chloride, polyvinylidene chloride, polyvinylidene fluoride or polytetrafluoroethylene; acrylic polymers such as polyacrylates, polymethacrylates or polyacrylonitrile; rubber; silicone polymers; phenol/formaldehyde resins; melamine/formaldehyde resins; urea/formaldehyde resins; epoxy resins; styrene butadiene rubber; acrylonitrile-butadiene rubber or chloroprene rubber; singly or in mixtures.

High molecular weight for purposes of the invention means an average molecular weight of from about 10² to about 10⁶ g/mole.

The pigment mixtures according to the invention can be added in any tinctorially effective amount to the high molecular weight organic material being pigmented. A pigmented substance composition comprising a high molecular weight organic material and from 0.01 to 80 % by weight, preferably from 0.1 to 30 % by weight, based on the high molecular weight organic material, of an pigment mixture according to the invention is advantageous. Concentrations of from 1 to 20 % by weight, especially of about 10 % by weight, can often be used in practice.

An ink according to the present invention comprises, as in the case of an ordinary printing ink, a pigment mixture, a binder, an auxiliary agent, and the like.

With respect to the binder resin, a thermoplastic resin may be used, examples of which include, polyethylene based polymers [polyethylene (PE), ethylene-vinyl acetate copolymer (EVA), vinyl chloride-vinyl acetate copolymer, vinyl alcohol-vinyl acetate copolymer], polypropylene (PP), vinyl based polymers [poly(vinyl chloride) (PVC), poly(vinyl butyral) (PVB), poly(vinyl alcohol) (PVA), poly(vinylidene chloride) (PVdC), poly(vinyl acetate) (PVAc), poly(vinyl formal) (PVF)], polystyrene based polymers [polystyrene (PS), styrene-acrylonitrile copolymer (AS), acrylonitrile-butadiene-styrene copolymer (ABS)], acrylic based polymers [poly(methyl methacrylate) (PMMA), MMA-styrene copolymer], polycarbonate (PC), celluloses [ethyl cellulose (EC),cellulose acetate (CA), propyl cellulose (CP), cellulose acetate butyrate (CAB), cellulose nitrate (CN)], fluorin based polymers [polychlorofluoroethylene (PCTFE), polytetrafluoroethylene (PTFE), tetrafluoroethylene-hexafluoroethylene copolymer (FEP), poly(vinylidene fluoride) (PVdF)], urethane based polymers (PU), nylons [type 6, type 66, type 610, type 11], polyesters (alkyl) [polyethylene terephthalate (PET), polybutylene terephthalate (PBT), polycyclohexane terephthalate (PCT)], novolac type phenolic resins, or the like. In addition, thermosetting resins such as resol type phenolic resin, a urea resin, a melamine resin, a polyurethane resin, an epoxy resin, an unsaturated polyester and the like, and natural resins such as protein, gum, shellac, copal, starch and rosin may also be used.

Further, the above resins may be in an emulsion form for use in a water-based paint. Emulsions for use in a water-based paint include for example, a vinyl acetate (homopolymer) emulsion, a vinyl acetate-acrylic ester copolymer emulsion, a vinyl acetate-ethylene copolymer emulsion (EVA emulsion), a vinyl acetate-vinyl versatate copolymer resin emulsion, a vinyl acetate-polyvinyl alcohol copolymer resin emulsion, a vinyl acetate-vinyl chloride copolymer resin emulsion, an acrylic emulsion, an acryl silicone emulsion, a styrene-acrylate copolymer resin emulsion, a polystyrene emulsion, an urethane polymer emulsion, a polyolefin chloride emulsion, an epoxy-acrylate dispersion, an SBR latex, and the like.

Furthermore, to the binder, a plasticizer for stabilizing the flexibility and strength of the print film and a solvent for adjusting the viscosity and drying property thereof may be added according to the needs therefor. A solvent of a low boiling temperature of about 100°C and a petroleum solvent of a high boiling temperature of 250°C or higher, may be used according to the type of the printing method. An alkylbenzene or the like, for example may be used as a solvent of a low boiling temperature.

Further in addition, an auxiliary agent including a variety of reactive agents for improving drying property, viscosity, and dispersibility, may suitably be added. The auxiliary agents are to adjust the performance of the ink, and for example, a compound that improves the abrasion resistance of the ink surface and a drying agent that accelerates the drying of the ink, and the like may be employed.

A photopolymerization-curable resin or an electron beam curable resin wherein a solvent is not used may also be employed as a binder resin that is a principal component of the vehicle. The examples thereof include an acrylic resin, and specific examples of acrylic monomers commercially available are shown below.

A monofunctional acrylate monomer that may be used includes for example, 2-ethylhexyl acrylate, 2-ethylhexyl-EO adduct acrylate, ethoxydiethylene glycol acrylate, 2-hydroxyethyl acrylate, 2-hydroxypropyl acrylate, 2-hydroxyethyl acrylate-caprolactone addduct, 2-phenoxyethyl acrylate, phenoxydiethylene glycol acrylate, nonyl phenol-EO adduct acrylate, (nonyl phenol-EO adduct)-caprolactone adduct acrylate, 2-hydroxy-3-phenoxypropyl acrylate, tetrahydrofurfuryl acrylate, furfuryl alcohol-caprolactone adduct acrylate, acryloyl morpholine, dicyclopentenyl acrylate, dicyclopentanyl acrylate, dicyclopentenyloxyethyl acrylate, isobornyl acrylate, (4,4-dimethyl-1,3-dioxane)-caprolactone adduct acrylate, (3-methyl-5,5-dimethyl-1,3-dioxane)-caprolactone adduct acrylate, and the like.

A polyfunctional acrylate monomer that may be used includes hexanediol diacrylate, neopentyl glycol diacrylate, polyethylene glycol diacrylate, tripropylene glycol diacrylate, neopentyl glycol hydroxypivalate diacrylate, (neopentyl glycol hydroxypivalate)-caprolactone adduct diacrylate, (1,6-hexanediol diglycidyl ether)-acrylic acid adduct, (hydroxypivalaldehyde-trimethylolpropane acetal) diacrylate, 2,2-bis[4-(acryloyloxydiethoxy)phenyl]propane, 2,2-bis[4-(acryloyloxydiethoxy)phenyl]methane; hydrogenated bisphenol A-ethylene oxide adduct diacrylate, tricyclodecanedimethanol diacrylate, trimethylolpropane triacrylate, pentaerithritol triacrylate, (trimethylolpropane-propylene oxide) adduct triacrylate, glycerine-propylene oxide adduct triacrylate, a mixture of dipentaerithritol hexaacrylate and pentaacrylate, esters of dipentaerithritol and lower fatty acid and acrylic acid, dipentaerithritol-caprolactone adduct acrylate, tris(acryloyloxyethyl) isocyanurate, 2-acryloyloxyethyl phosphate, and the like.

Inks comprising the above resins are free of solvent and are so constituted as to polymerize in chain reaction upon irradiation by an electron beam or electromagnetic waves.

With respect to inks of ultraviolet-irradiation type among these inks, a photopolymerization initiator, and depending on the needs therefor, a sensitizing agent, and auxiliary agents such as a polymerization inhibitor and a chain transfer agent, and the like may be added thereto.

With respect to photo-polymerization initiators, there are, (1) an initiator of direct photolysis type including an arylalkyl ketone, an oxime ketone, an acylphosphine oxide, or the like, (2) an initiator of radical polymerization reaction type including a benzophenone derivative, a thioxanthone derivative, or the like, (3) an initiator of cationic polymerization reaction type including an aryl diazonium salt, an aryl iodinium salt, an aryl sulfonium salt, and an aryl acetophenone salt, or the like, and in addition, (4) an initiator of energy transfer type, (5) an initiator of photoredox type, (6) an initiator of electron transfer type, and the like. With respect to the inks of electron beam-curable type, a photopolymerization initiator is not necessary and a resin of the same type as in the case of the ultraviolet-irradiation type inks can be used, and various kinds of auxiliary agent may be added thereto according to the needs therefor.

The inks comprise a total content of pigment mixture of from 0.1 to 25 % by weight, preferably 0.4-22 % by weight, most preferred 0.4-16.5 % by weight based on the total weight of the ink. Component A is preferably contained in an amount of from 0.2 to 2.0 % by weight, most preferably of from 0.2 to 1.5 % by weight based on the total weight of the ink. Component B is preferably contained in an amount of from 0.2 to 20 % by weight, most preferably of from 0.2 to 15 % by weight based on the total weight of the ink.

In particular the component A and B mixtures may be incorporated into skin-care products, bath and shower additives, preparations containing fragrances and odoriferous substances, hair-care products, deodorizing and antiperspirant preparations, decorative preparations, light protection formulations and preparations containing active ingredients and uses thereof to achieve special color effects.

Body-care products are, in particular, skin-care products, such as body oils, body lotions, body gels, treatment creams, skin protection ointments, shaving preparations, such as shaving foams or gels, skin powders, such as baby powder, moisturizing gels, moisturizing sprays, revitalizing body sprays, cellulite gels and peeling preparations.

In a preferred embodiment of the present invention the personal care product is a body-care product for the skin and its adnexa.

Suitable bath and shower additives are shower gels, bath-salts, bubble baths and soaps.

Preparations containing fragrances and odoriferous substances are in particular scents, perfumes, toilet waters and shaving lotions (aftershave preparations).

Suitable hair-care products are, for example, shampoos for humans and animals, in particular dogs, hair conditioners, products for styling and treating hair, perming agents, hair sprays and lacquers, hair gels, hair fixatives and hair dyeing or bleaching agents.

Suitable decorative preparations are in particular lipsticks, nail varnishes, eye shadows, mascaras, dry and moist make-up, rouge, powders, depilatory agents and suntan lotions.

Suitable cosmetic formulations containing active ingredients are in particular hormone preparations, vitamin preparations and vegetable extract preparations.

The mentioned body-care products may be in the form of creams, ointments, pastes, foams, gels, lotions, powders, make-ups, sprays, sticks or aerosols.

The present invention therefore also relates to a body-care product comprising components A and B.

The mixture of components A and B are present in the body care and household products in a concentration of about 0.0001 % to about 25%, based on the total formulation, preferably from about 0.001 % to about 15%, and most preferably from about 0.05% to about 10%.

The present pigment mixtures are particularly suitable for coloration of cosmetic and body care products, in particular:
- skin-care preparations, e.g. skin-washing and cleansing preparations in the form of tablet-form or liquid soaps, soapless detergents or washing pastes,
- bath preparations, e.g. liquid (foam baths, milks, shower preparations) or solid bath preparations, e.g. bath cubes and bath salts;
- skin-care preparations, e.g. skin emulsions, multi-emulsions or skin oils; body oils, body lotions, body gels; skin protection ointments;
- cosmetic personal care preparations, e.g. facial make-up in the form of day creams or powder creams, face powder (loose or pressed), rouge or cream make-up, eye-care preparations, e.g. eyeshadow preparations, mascara, eyeliner, eye creams or eye-fix creams; lip-care preparations, e.g. lipsticks, lip gloss, lip contour pencils, nail-care preparations, such as nail varnish, nail varnish removers, nail hardeners or cuticle removers;
- foot-care preparations, e.g. foot baths, foot powders, foot creams or foot balsams, special deodorants and antiperspirants or callus-removing preparations;
- light-protective preparations, such as sun milks, lotions, creams or oils, sunblocks or tropicals, pre-tanning preparations or after-sun preparations;
- skin-tanning preparations, e.g. self-tanning creams;
- depigmenting preparations, e.g. preparations for bleaching the skin or skin-lightening preparations;
- insect-repellents, e.g. insect-repellent oils, lotions, sprays or sticks;
- deodorants, such as deodorant sprays, pump-action sprays, deodorant gels, sticks or roll-ons;
- antiperspirants, e.g. antiperspirant sticks, creams or roll-ons;
- preparations for cleansing and caring for blemished skin, e.g. synthetic detergents (solid or liquid), peeling or scrub preparations or peeling masks;
- hair-removal preparations in chemical form (depilation), e.g. hair-removing powders, liquid hair-removing preparations, cream- or paste-form hair-removing preparations, hair-removing preparations in gel form or aerosol foams;
- shaving preparations, e.g. shaving soap, foaming shaving creams, non-foaming shaving creams, foams and gels, preshave preparations for dry shaving, aftershaves or aftershave lotions;
- fragrance preparations, e.g. fragrances and odoriferous substances containing preparations (scents, eau de Cologne, eau de toilette, eau de parfum, parfum de toilette, perfume), perfume oils or perfume creams;
- cosmetic hair-treatment preparations, e.g. hair-washing preparations in the form of shampoos and conditioners, hair-care preparations, e.g. pretreatment preparations, hair tonics, styling creams, styling gels, pomades, hair rinses, treatment packs, intensive hair treatments, hair-structuring preparations, e.g. hair-waving preparations for permanent waves (hot wave, mild wave, cold wave), hair-straightening preparations, liquid hair-setting preparations, hair foams, hairsprays, bleaching preparations, e.g. hydrogen peroxide solutions, lightening shampoos, bleaching creams, bleaching powders, bleaching pastes or oils, temporary, semi-permanent or permanent hair colourants, preparations containing self-oxidising dyes, or natural hair colourants, such as henna or camomile;
- decorative preparations, in particular lipsticks, nail varnishes, eye shadows, mascaras, dry and moist make-up, rouge, powders, depilatory agents and suntan lotions
- cosmetic formulations containing active ingredients, in particular hormone preparations, vitamin preparations, vegetable extract preparations and antibacterial preparations.

### Presentation forms

The final formulations containing the pigment mixtures may exist in a wide variety of presentation forms, for example:
- in the form of liquid preparations as a W/O, O/W, O/W/O, W/O/W or PIT emulsion and all kinds of microemulsions,
- in the form of a gel,
- in the form of an oil, a cream, milk or lotion,
- in the form of a stick,
- in the form of a spray (spray with propellent gas or pump-action spray) or an aerosol,
- in the form of a foam, or
- in the form of a paste.

Examples of body care products of the present invention are listed in the Table below:

| Body care product | Ingredients |
|---|---|
| moisturizing cream | vegetable oil, emulsifier, thickener, perfume, water, stabilizers, preservatives, dyes/pigments |
| Shampoo | surfactant, emulsifier, preservatives, perfume, antioxidant, UV absorbers, dyes/pigments |
| Lipstick | vegetable oils, waxes, stabilizers, dyes/pigments |
| eye shadow | Talc, Zinc Stearate, oils, stabilizers, pigments |
| Makeup | Water, thickener, oils, emulsifier, perfume, preservatives, stabilizers, pigments |

Various features and aspects of the present invention are illustrated further in the examples that follow. While these examples are presented to show one skilled in the art how to operate within the scope of this invention, they are not to serve as a limitation on the scope of the invention where such scope is only defined in the claims. Unless otherwise indicated in the following examples and elsewhere in the specification and claims, all parts and percentages are by weight, temperatures are in degrees centigrade and pressures are at or near atmospheric.

### EXAMPLES

### Example 1

a) One kilogram of vinylketone type clear varnish is prepared by mild stirring at 3000 rpm for 30 min at room temperature of a formulation containing 100g 1-ethoxypropanol, 760g methylethylketone and 140g VMCH (UCC).
b) A vinylketone ink is prepared by dispersing in a Skandex^{®} for 2 hours in a 400 ml glass bottle 1.5 parts of compound A and 230g of glass beads of 2mm diameter into 98.5 parts of the clear varnish prepared according to example 1a). Centrifugation and removal of the glass beads affords a concentrate of compound A.
c) A concentrate of MICROLITH^{®} DPP Red B-K is prepared by mild stirring with Dispermat at 6000 rpm for 20 min in a 400ml glass bottle of 12 parts of MICROLITH DPP Red B-K, 5 parts of Vinylite VYHH from Union Carbide, 10 parts of methoxypropylacetate and 73 parts of isobutylmethylketone.
d) 10 parts of the concentrate of example 1c) are stirred into 90 parts of the concentrate of example 1b). The thus obtained homogeneous dispersion is applied by hand-coater (50 µm wet film thickness) on contrast paper (black and white) providing an opaque bordeaux-red grey print with sparkling metallic effect depending on viewing angle.

Compound A are graphite nanoplatelets, the production of which is described in Example 1 (plasma expansion) and 4 (subsequent exfoliation (sonication)) of PCT/EP2009/052127: - An expandable graphite powder (Grafguard® 220-80N) is fed at a rate of 2 kg/hour into a plasma reactor with a Tekna PL-70 plasma torch operated at a power of 80 kW. The sheath gas is 150 slpm argon [slpm = standard liters per minute; standard conditions for the calculation of slpm are defined as: Tn 0°C (32°F), Pn = 1.01 bara (14.72 psi)] and the central gas is argon at 40 slpm. The operating pressure is maintained at slightly lower than atmospheric pressure (93 325,6579 Pa = 700 torr). An injection probe designed for powder injection with dispersion is positioned to allow for maximum expansion without significant vaporization of the graphite flakes. The expanded flakes are collected in a filter after passing a heat exchange zone.
- Ultrasonication is used to exfoliate plasma-expanded graphite and create a stable dispersion in water or non-aqueous liquids. Into a 2-liter flask, 1.5 liters of liquid are added. If the liquid is mineral oil, no dispersant is required. For aqueous dispersions, 4 g of PLURONIC P123 is added to 1.5 L of water. For toluene, 4 g of Efka 6220 is added (fatty acid modified polyester). The mixture is stirred until dissolved. Gentle heat is applied if necessary. 4.0 g of plasma-expanded graphite is added to the 1.5 L of liquid. The contents are then stirred in order to initially wet the expanded graphite which tends to float on top of the liquid. With the aid of a 750-watt ultrasonic processor (VCX 750 Sonics & Materials, Inc.), the liquid/graphite mixture is ultrasonicated @ 40% intensity for a total of 40 minutes. A pulse method (10 seconds ON - 10 seconds OFF) is used to prevent over heating. During the ultrasonic treatment, a noticeable reduction in particle size is observed and particles become suspended (no settling occurs upon standing). If a solid material is desired, the dispersion is vacuum filtered using a WHATMAN #1 paper filter. The filter cake from mineral oil contains 85 wt % mineral oil and 15 wt % graphite, where as the toluene and water filter cakes contain almost 90 wt % liquid, 8 wt % graphite and 2 wt % residual dispersant.
As described in Example 5 of PCT/EP2009/052127 controlled cavitation may be used instead of ultrasonication for the exfoilation of the graphite.

### Example 2

10 parts of the concentrate of example 1c) and 2.5 parts of XYMARA^{®} Silver Pearl S23 are stirred into to 40 parts of the concentrate of example 1b). The thus obtained homogeneous dispersion is applied by hand-coater (50 µm wet film thickness) on contrast paper (black and white) providing an opaque reddish grey print with silver metallic effect.

### Example 3

A vinylketone ink is prepared by dispersing in a Skandex^{®} for 2 hours in a 400 ml glass bottle 1.5 parts of compound A and 230g of glass beads of 2mm diameter into 93.5 parts of the clear varnish prepared according to example 1a).
After centrifugation and removal of the glass beads, 5 parts of XYMARA^{®} Silver Pearl S23 are added and mildly stirred providing a homogeneous dispersion which is applied by hand-coater (50 µm wet film thickness) on contrast paper (black and white) and results in an opaque silver metallic effect.

### Example 4

A vinylketone ink is prepared by dispersing in a Skandex^{®} for 2 hours in a 400 ml glass bottle 1.5 parts of compound A and 230g of glass beads of 2mm diameter into 88.5 parts of the clear varnish prepared according to example 1a).
After centrifugation and removal of the glass beads, 10 parts of XYMARA^{®} Silver Pearl S23 are added and mildly stirred providing a homogeneous dispersion which is applied by hand-coater (50 µm wet film thickness) on contrast paper (black and white) and results in an opaque silver metallic effect.

### Example 5

A vinylketone ink is prepared by dispersing in a Skandex^{®} for 2 hours in a 400 ml glass bottle 1.5 parts of compound A and 230g of glass beads of 2mm diameter into 83.5 parts of the clear varnish prepared according to example 1a).
After centrifugation and removal of the glass beads, 15 parts of XYMARA^{®} Silver Pearl S23 are added and mildly stirred providing a homogeneous dispersion which is applied by hand-coater (50 µm wet film thickness) on contrast paper (black and white) and results in an opaque silver metallic effect.
Measurements performed with Datacolor Multi FX 10 apparatus on the print of example 5 (on the white part of the contrast paper) are represented in the table below.

| **Angles** | **L*** | **a*** | **b*** |
|---|---|---|---|
| **25°/170°** | **135.56** | **-1.77** | **-5.90** |
| **25°/140°** | **141.61** | **-1.43** | **-5.49** |
| **45°/150°** | **140.44** | **-1.64** | **-5.84** |
| **45°/120°** | **136.73** | **-1.72** | **-4.36** |
| **75°/120°** | **128.20** | **-1.85** | **-3.96** |
| **75°/90°** | **123.41** | **-1.56** | **-2.80** |
| **45°/110°** | **115.33** | **-1.23** | **-1.41** |
| **45°/90°** | **88.92** | **-0.32** | **2.84** |
| **45°/60_{°}** | **77.00** | **0.25** | **5.39** |
| **45°/25°** | **77.18** | **-0.19** | **5.26** |

### Example 6

A vinylketone ink is prepared by dispersing in a Skandex^{®} for 2 hours in a 400 ml glass bottle 1.5 parts of compound A and 230g of glass beads of 2mm diameter into 93.5 parts of the clear varnish prepared according to example 1a).

After centrifugation and removal of the glass beads, 5 parts of XYMARA^{®} Bronze Pearl B04 are added and mildly stirred providing a homogeneous dispersion which is applied by hand-coater (50 µm wet film thickness) on contrast paper (black and white) and results in an opaque bronze metallic effect.

### Example 7

A vinylketone ink is prepared by dispersing in a Skandex^{®} for 2 hours in a 400 ml glass bottle 1.5 parts of compound A and 230g of glass beads of 2mm diameter into 88.5 parts of the clear varnish prepared according to example 1a).
After centrifugation and removal of the glass beads, 10 parts of XYMARA^{®} Bronze Pearl B04 are added and mildly stirred providing a homogeneous dispersion which is applied by hand-coater (50 µm wet film thickness) on contrast paper (black and white) and results in an opaque bronze metallic effect.

### Example 8

A vinylketone ink is prepared by dispersing in a Skandex^{®} for 2 hours in a 400 ml glass bottle 1.5 parts of compound A and 230g of glass beads of 2mm diameter into 83.5 parts of the clear varnish prepared according to example 1a).
After centrifugation and removal of the glass beads, 15 parts of XYMARA^{®} Bronze Pearl B04 are added and mildly stirred providing a homogeneous dispersion which is applied by hand-coater (50 µm wet film thickness) on contrast paper (black and white) and results in an opaque bronze metallic effect.
Measurements performed with Datacolor Multi FX 10 apparatus on the print of example 8 (on the white part of the contrast paper) are represented in the table below.

| **Angles** | **L*** | **a*** | **b*** |
|---|---|---|---|
| **25°/170°** | **79.17** | **8.07** | **-1.00** |
| **25°/140°** | **98.41** | **22.94** | **1.73** |
| **45°/150°** | **95.93** | **23.54** | **1.62** |
| **45°/120°** | **89.97** | **25.33** | **2.10** |
| **75°/120°** | **79.54** | **22.30** | **0.60** |
| **75°/90°** | **71.82** | **21.39** | **1.17** |
| **45°/110°** | **54.18** | **14.43** | **1.90** |
| **45°/90°** | **26.58** | **6.89** | **1.28** |
| **45°/60°** | **13.89** | **5.57** | **1.75** |
| **45°/25°** | **13.37** | **4.45** | **0.86** |

### Example 9

A vinylketone ink is prepared by dispersing in a Skandex^{®} for 2 hours in a 400 ml glass bottle 1.5 parts of compound A and 230g of glass beads of 2mm diameter into 93.5 parts of the clear varnish prepared according to example 1a).
After centrifugation and removal of the glass beads, 5 parts of XYMARA^{®} Gold Pearl G03 are added and mildly stirred providing a homogeneous dispersion which is applied by hand-coater (50 µm wet film thickness) on contrast paper (black and white) and results in an opaque gold metallic effect.

### Example 10

A vinylketone ink is prepared by dispersing in a Skandex^{®} for 2 hours in a 400 ml glass bottle 1.5 parts of compound A and 230g of glass beads of 2mm diameter into 88.5 parts of the clear varnish prepared according to example 1a).
After centrifugation and removal of the glass beads, 10 parts of XYMARA^{®} Gold Pearl G03 are added and mildly stirred providing a homogeneous dispersion which is applied by hand-coater (50 µm wet film thickness) on contrast paper (black and white) and results in an opaque gold metallic effect.

### Example 11

A vinylketone ink is prepared by dispersing in a Skandex^{®} for 2 hours in a 400 ml glass bottle 1.5 parts of compound A and 230g of glass beads of 2mm diameter into 83.5 parts of the clear varnish prepared according to example 1a).
After centrifugation and removal of the glass beads, 15 parts of XYMARA^{®} Gold Pearl G03 are added and mildly stirred providing a homogeneous dispersion which is applied by hand-coater (50 µm wet film thickness) on contrast paper (black and white) and results in an opaque gold metallic effect.
Measurements performed with Datacolor Multi FX 10 apparatus on the print of example 11 (on the white part of the contrast paper) are represented in the table below.

| **Angles** | **L*** | **a*** | **b*** |
|---|---|---|---|
| **25°/170°** | **130.03** | **-0.26** | **10.86** |
| **25°/140°** | **128.25** | **2.12** | **28.44** |
| **45°/150°** | **117.11** | **2.88** | **29.14** |
| **45°/120°** | **107.38** | **5.14** | **34.27** |
| **75°/120°** | **92.59** | **5.71** | **29.88** |
| **75°/90°** | **85.00** | **5.65** | **27.82** |
| **45°/110°** | **65.12** | **2.55** | **16.37** |
| **45°/90°** | **33.38** | **1.16** | **6.45** |
| **45°/60°** | **19.40** | **0.90** | **5.06** |
| **45°/25°** | **16.26** | **1.39** | **4.69** |

### Example 12

A vinylketone ink is prepared by dispersing in a Skandex^{®} for 2 hours in a 400 ml glass bottle 1.5 parts of compound A and 230g of glass beads of 2mm diameter into 93.5 parts of the clear varnish prepared according to example 1a).
After centrifugation and removal of the glass beads, 5 parts of XYMARA^{®} Gold Pearl G23 are added and mildly stirred providing a homogeneous dispersion which is applied by hand-coater (50 µm wet film thickness) on contrast paper (black and white) and results in an opaque gold metallic effect.

### Example 13

A vinylketone ink is prepared by dispersing in a Skandex^{®} for 2 hours in a 400 ml glass bottle 1.5 parts of compound A and 230g of glass beads of 2mm diameter into 88.5 parts of the clear varnish prepared according to example 1a).
After centrifugation and removal of the glass beads, 10 parts of XYMARA^{®} Gold Pearl G23 are added and mildly stirred providing a homogeneous dispersion which is applied by hand-coater (50 µm wet film thickness) on contrast paper (black and white) and results in an opaque gold metallic effect.

### Example 14

A vinylketone ink is prepared by dispersing in a Skandex^{®} for 2 hours in a 400 ml glass bottle 1.5 parts of compound A and 230g of glass beads of 2mm diameter into 83.5 parts of the clear varnish prepared according to example 1a).
After centrifugation and removal of the glass beads, 15 parts of XYMARA^{®} Gold Pearl G23 are added and mildly stirred providing a homogeneous dispersion which is applied by hand-coater (50 µm wet film thickness) on contrast paper (black and white) and results in an opaque gold metallic effect.

### Example 15

A vinylketone ink is prepared by dispersing in a Skandex^{®} for 2 hours in a 400 ml glass bottle 1.5 parts of compound A and 230g of glass beads of 2mm diameter into 93.5 parts of the clear varnish prepared according to example 1a).
After centrifugation and removal of the glass beads, 5 parts of XYMARA^{®} Dual Pearl D05 are added and mildly stirred providing a homogeneous dispersion which is applied by hand-coater (50 µm wet film thickness) on contrast paper (black and white) and results in an opaque gold metallic effect.

### Example 16

A vinylketone ink is prepared by dispersing in a Skandex^{®} for 2 hours in a 400 ml glass bottle 1.5 parts of compound A and 230g of glass beads of 2mm diameter into 88.5 parts of the clear varnish prepared according to example 1a).
After centrifugation and removal of the glass beads, 10 parts of XYMARA^{®} Dual Pearl D05 are added and mildly stirred providing a homogeneous dispersion which is applied by hand-coater (50 µm wet film thickness) on contrast paper (black and white) and results in an opaque gold metallic effect.

### Example 17

A vinylketone ink is prepared by dispersing in a Skandex^{®} for 2 hours in a 400 ml glass bottle 1.5 parts of compound A and 230g of glass beads of 2mm diameter into 83.5 parts of the clear varnish prepared according to example 1a).
After centrifugation and removal of the glass beads, 15 parts of XYMARA^{®} Dual Pearl D05 are added and mildly stirred providing a homogeneous dispersion which is applied by hand-coater (50 µm wet film thickness) on contrast paper (black and white) and results in an opaque gold metallic effect.

### Example 18

A vinylketone ink is prepared by dispersing in a Skandex^{®} for 2 hours in a 400 ml glass bottle 1.5 parts of compound A and 230g of glass beads of 2mm diameter into 93.5 parts of the clear varnish prepared according to example 1a).
After centrifugation and removal of the glass beads, 5 parts of XYMARA^{®} Dual Pearl D19 are added and mildly stirred providing a homogeneous dispersion which is applied by hand-coater (50 µm wet film thickness) on contrast paper (black and white) and results in an opaque violet metallic effect.

### Example 19

A vinylketone ink is prepared by dispersing in a Skandex^{®} for 2 hours in a 400 ml glass bottle 1.5 parts of compound A and 230g of glass beads of 2mm diameter into 88.5 parts of the clear varnish prepared according to example 1a).
After centrifugation and removal of the glass beads, 10 parts of XYMARA^{®} Dual Pearl D19 are added and mildly stirred providing a homogeneous dispersion which is applied by hand-coater (50 µm wet film thickness) on contrast paper (black and white) and results in an opaque violet metallic effect.

### Example 20

A vinylketone ink is prepared by dispersing in a Skandex^{®} for 2 hours in a 400 ml glass bottle 1.5 parts of compound A and 230g of glass beads of 2mm diameter into 83.5 parts of the clear varnish prepared according to example 1 a).
After centrifugation and removal of the glass beads, 15 parts of XYMARA^{®} Dual Pearl D19 are added and mildly stirred providing a homogeneous dispersion which is applied by hand-coater (50 µm wet film thickness) on contrast paper (black and white) and results in an opaque violet metallic effect.

### Example 21

A vinylketone ink is prepared by dispersing in a Skandex^{®} for 2 hours in a 400 ml glass bottle 1.5 parts of compound A and 230g of glass beads of 2mm diameter into 93.5 parts of the clear varnish prepared according to example 1a).
After centrifugation and removal of the glass beads, 5 parts of XYMARA^{®} Dual Pearl D21 are added and mildly stirred providing a homogeneous dispersion which is applied by hand-coater (50 µm wet film thickness) on contrast paper (black and white) and results in an opaque blue metallic effect.

### Example 22

A vinylketone ink is prepared by dispersing in a Skandex^{®} for 2 hours in a 400 ml glass bottle 1.5 parts of compound A and 230g of glass beads of 2mm diameter into 88.5 parts of the clear varnish prepared according to example 1a).
After centrifugation and removal of the glass beads, 10 parts of XYMARA^{®} Dual Pearl D21 are added and mildly stirred providing a homogeneous dispersion which is applied by hand-coater (50 µm wet film thickness) on contrast paper (black and white) and results in an opaque blue metallic effect.

### Example 23

A vinylketone ink is prepared by dispersing in a Skandex^{®} for 2 hours in a 400 ml glass bottle 1.5 parts of compound A and 230g of glass beads of 2mm diameter into 83.5 parts of the clear varnish prepared according to example 1a).
After centrifugation and removal of the glass beads, 15 parts of XYMARA^{®} Dual Pearl D21 are added and mildly stirred providing a homogeneous dispersion which is applied by hand-coater (50 µm wet film thickness) on contrast paper (black and white) and results in an opaque blue metallic effect.
Measurements performed with Datacolor Multi FX 10 apparatus on the print of example 23 (on the white part of the contrast paper) are represented in the table below.

| **Angles** | **L*** | **a*** | **b*** |
|---|---|---|---|
| **25°/170°** | **115.66** | **3.68** | **-15.57** |
| **25°/140°** | **113.41** | **3.15** | **-34.28** |
| **45°/150°** | **102.82** | **1.98** | **-34.24** |
| **45°/120°** | **95.88** | **-2.66** | **-36.17** |
| **75°/120°** | **83.49** | **-5.70** | **-31.07** |
| **75°/90°** | **80.74** | **-7.17** | **-28.56** |
| **45°/110°** | **66.11** | **-2.25** | **-20.13** |
| **45°/90°** | **36.98** | **-0.82** | **-7.53** |
| **45°/60°** | **23.07** | **0.08** | **-4.09** |
| **45°/25°** | **20.75** | **0.29** | **-4.99** |

### Example 24

A vinyl ketone ink is prepared by dispersing in a Skandex^{®} for 2 hours in a 400 ml glass bottle 1.5 parts of compound A and 230g of glass beads of 2mm diameter into 93.5 parts of the clear varnish prepared according to example 1a).
After centrifugation and removal of the glass beads, 5 parts of XYMARA^{®} Dual Pearl D31 are added and mildly stirred providing a homogeneous dispersion which is applied by hand-coater (50 µm wet film thickness) on contrast paper (black and white) and results in an opaque green metallic effect.

### Example 25

A vinylketone ink is prepared by dispersing in a Skandex^{®} for 2 hours in a 400 ml glass bottle 1.5 parts of compound A and 230g of glass beads of 2mm diameter into 88.5 parts of the clear varnish prepared according to example 1a).

After centrifugation and removal of the glass beads, 10 parts of XYMARA^{®} Dual Pearl D31 are added and mildly stirred providing a homogeneous dispersion which is applied by hand-coater (50 µm wet film thickness) on contrast paper (black and white) and results in an opaque green metallic effect.

### Example 26

A vinylketone ink is prepared by dispersing in a Skandex^{®} for 2 hours in a 400 ml glass bottle 1.5 parts of compound A and 230g of glass beads of 2mm diameter into 83.5 parts of the clear varnish prepared according to example 1a).
After centrifugation and removal of the glass beads, 15 parts of XYMARA^{®} Dual Pearl D31 are added and mildly stirred providing a homogeneous dispersion which is applied by hand-coater (50 µm wet film thickness) on contrast paper (black and white) and results in an opaque green metallic effect.
Measurements performed with Datacolor Multi FX 10 apparatus on the print of example 26 (on the white part of the contrast paper) are represented in the table below.

| **Angles** | **L*** | **a*** | **b*** |
|---|---|---|---|
| **25°/170°** | **119.07** | **-7.21** | **-3.91** |
| **25°/140°** | **123.79** | **-16.99** | **-0.36** |
| **45°/150°** | **114.76** | **-17.65** | **1.27** |
| **45°/120°** | **108.03** | **-19.58** | **7.05** |
| **75°/120°** | **94.81** | **-17.91** | **9.68** |
| **75°/90°** | **90.44** | **-16.58** | **10.79** |
| **45°/110°** | **74.39** | **-11.52** | **4.02** |
| **45°/90°** | **41.17** | **-4.13** | **-0.04** |
| **45°/60°** | **25.34** | **-0.88** | **-3.43** |
| **45°/25°** | **23.28** | **-0.97** | **-4.82** |

### Example 27

A vinyl ketone ink is prepared by dispersing in a Skandex^{®} for 2 hours in a 400 ml glass bottle 1.5 parts of compound A and 230g of glass beads of 2mm diameter into 83.5 parts of the clear varnish prepared according to example 1a).
After centrifugation and removal of the glass beads, 15 parts of 2,9-dichloro-quinacridone in platelet form, which is described in WO08/055807, are added and mildly stirred providing a homogeneous dispersion which is applied by hand-coater (50 µm wet film thickness) on contrast paper (black and white) and results in an opaque red-bronze metallic effect.

### Example 28

a) A nitrocellulose type clear varnish is prepared by mild stirring at 500 rpm for 30 min at room temperature of a formulation containing 14 parts DLX 3-5 IPA (Low Nitrogen Grade 10.7% -11.2%) from Nobel Enterprises and 86 parts ethylacetate.
b) A nitrocellulose ink is prepared by dispersing in a Skandex^{®} for 2 hours in a 400 ml glass bottle 1.5 parts of compound A and 230g of glass beads of 2mm diameter into 98.5 parts of the clear varnish prepared according to example 28a). Centrifugation and removal of the glass beads affords a nitrocellulose concentrate containing compound A.
c) Mild stirring of 5 parts of XYMARA^{®} Nordic Frost into 95 parts of the concentrate prepared in example 32 followed by application by hand-coater (50 µm wet film thickness) on contrast paper (black and white) and results in an opaque sparkling grey print.

### Example 29

Mild stirring of 10 parts of BAYFERROX 110M into 90 parts of the concentrate prepared in example 28b) followed by application by hand-coater (50 µm wet film thickness) on contrast paper (black and white) and results in an opaque brownish print with mild sparkling effect depending on viewing angle.

### Example 30

Formulations of example 2 to example 26 are applied on contrast paper by screen-printing using a screen with characteristics 43-80.

### Example 31

All formulations are also printed on transparent substrates such as for example Melinex (polyester) with which the described effect is visible both by direct viewing (side of the print) and by reverse viewing (back of the print).

### Comparative Example 1 - GRAPHITAN 7525 & XYMARA^{®} Silver Pearl S23

A vinyl ketone ink is prepared by dispersing in a Skandex^{®} for 2 hours in a 400 ml glass bottle 1.5 g of GRAPHITAN 7525 from CIBA and 230g of glass beads of 2mm diameter into 88.5 g of the clear varnish prepared according to example 1a). After centrifugation and removal of the glass beads, 10 g of XYMARA^{®} Silver Pearl S23 are added and mildly stirred providing a homogeneous dispersion which is applied by hand-coater (50 µm wet film thickness) on contrast paper (black and white) and results in an non-opaque silver print in comparison to the print obtained in example 4.

### Comparative Example 2 - GRAPHITAN 7525 & XYMARA^{®} Bronze Pearl B03

A vinylketone ink is prepared by dispersing in a Skandex^{®} for 2 hours in a 400 ml glass bottle 1.5 g of GRAPHITAN 7525 from CIBA and 230g of glass beads of 2mm diameter into 88.5 g of the clear varnish prepared according to example 1 a). After centrifugation and removal of the glass beads, 10 g of XYMARA^{®} Bronze Pearl B03 are added and mildly stirred providing a homogeneous dispersion which is applied by hand-coater (50 µm wet film thickness) on contrast paper (black and white) and results in an non-opaque bronze print in comparison to the print obtained in example 7.

### Comparative Example 3 - GRAPHITAN 7525 & XYMARA^{®} Gold Pearl G03

A vinyl ketone ink is prepared by dispersing in a Skandex^{®} for 2 hours in a 400 ml glass bottle 1.5 g of GRAPHITAN 7525 from CIBA and 230g of glass beads of 2mm diameter into 88.5 g of the clear varnish prepared according to example 1a). After centrifugation and removal of the glass beads, 10 g of XYMARA^{®} Gold Pearl G03 are added and mildly stirred providing a homogeneous dispersion which is applied by hand-coater (50 µm wet film thickness) on contrast paper (black and white) and results in an non-opaque gold print in comparison to the print obtained in example 10.

### Comparative Example 4 - MICROLITH^{®} Black C-K & XYMARA^{®} Silver Pearl S23

a) 10 parts of DOWANOL PMA (Propyleneglycol Monomethylether Acetate), 5 parts of Vinylite VYHH and 73 parts of Isobutylmethylketone are stirred with Dispermat at 3000rpm for 30 min. Addition of 12 parts MICROLITH^{®} Black C-K and further stirring at 1500rpm for 20 min affords a concentrate of C.I. Pigment Black 7.
b) A vinylketone ink is prepared by dispersing in a Skandex^{®} for 2 hours in a 400 ml glass bottle 25 g of concentrate prepared in Comparative Example 4a) and 230g of glass beads of 2mm diameter into 65 g of the clear varnish prepared according to example 1a). After centrifugation and removal of the glass beads, 10 g of XYMARA^{®} Silver Pearl S23 are added and mildly stirred providing a homogeneous dispersion which is applied by hand-coater (50 µm wet film thickness) on contrast paper (black and white) and results in an opaque black print with no silver coloration in comparison to the print obtained in example 4.

### Comparative Example 5 - MICROLITH^{®} Black C-K & XYMARA^{®} Bronze Pearl B03

A vinylketone ink is prepared by dispersing in a Skandex^{®} for 2 hours in a 400 ml glass bottle 25 g of concentrate prepared in Comparative Example 4a) and 230g of glass beads of 2mm diameter into 65 g of the clear varnish prepared according to example 1a). After centrifugation and removal of the glass beads, 10 g of XYMARA^{®} Bronze Pearl B03 are added and mildly stirred providing a homogeneous dispersion which is applied by hand-coater (50 µm wet film thickness) on contrast paper (black and white) and results in an opaque black print with very slight bronze coloration in comparison to the print obtained in example 7.

### Comparative Example 6 - MICROLITH^{®} Black C-K & XYMARA^{®} Gold Pearl G03

A vinyl ketone ink is prepared by dispersing in a Skandex^{®} for 2 hours in a 400 ml glass bottle 25 g of concentrate prepared in Comparative Example 4a) and 230g of glass beads of 2mm diameter into 65 g of the clear varnish prepared according to example 1a). After centrifugation and removal of the glass beads, 10 g of XYMARA^{®} Gold Pearl G03 are added and mildly stirred providing a homogeneous dispersion which is applied by hand-coater (50 µm wet film thickness) on contrast paper (black and white) and results in an opaque black print with very slight gold coloration in comparison to the print obtained in example 13.

### Example 32

a) One kilogram of nitrocellulose clear varnish is prepared by mild stirring at 3000rpm for 30min at room temperature of a formulation containing 100g AH27 (20%ATBC, Christ Chemie AG Reinach), 60g JONCRYL 68 (BASF), 100g 1-ethoxypropanol, 200g ethyl acetate and 540g ethanol.
b) A nitrocellulose ink is prepared by dispersing in a Skandex^{®} for 2 hours in a 400 ml glass bottle 12 parts of compound A described in example 4 of PCT/EP2009/052127 where the oil is HONEYWELL AC 617A (80%) and 230g of glass beads of 2mm diameter into 68 parts of the clear varnish prepared according to example 32a). Centrifugation and removal of the glass beads afford a concentrate of compound A.

### Example 33

A mixture of 35 parts of the varnish of example 32a) and 15 parts of XYMARA^{®} Silver Pearl S23 is gently stirred. 50 parts of the concentrate of example 32b) are added to this dispersion and the thus obtained homogeneous ink has a viscosity of 59 sec (DIN Cup N°4).

### Example 33a

Application by hand-coater (50µm wet film thickness) of the ink of example 33 on AMCOR cardboard provides an opaque silver metallic effect with coat weight of 15g/m². Measurements performed with Datacolor Multi FX 10 apparatus on the print of example 33a are represented in the table below.

| **Angles** | **L*** | **a*** | **b*** |
|---|---|---|---|
| **25°/170°** | **115.90** | **-0.06** | **-4.73** |
| **25°1140°** | **125.39** | **-1.17** | **-11.60** |
| **45°/150°** | **121.94** | **-1.27** | **-11.76** |
| **45°/120°** | **113.21** | **-1.24** | **-11.00** |
| **75°/120°** | **102.96** | **-1.54** | **-10.28** |
| **75°/90°** | **97.29** | **-1.56** | **-9.63** |
| **45°/110°** | **85.99** | **-0.93** | **-7.40** |
| **45°/90°** | **50.48** | **-0.59** | **-3.35** |
| **45°/60°** | **31.03** | **-0.43** | **-2.82** |
| **45°/25°** | **25.77** | **-0.47** | **-4.38** |

### Example 33b

Application by hand-coater (50µm wet film thickness) of the ink of example 33 on PVC wallpaper provides an opaque silver metallic effect.

### Example 33c

Application by hand-coater (50µm wet film thickness) of the ink of example 33 on MELINEX 505 foil (thickness 75µm) provides an opaque silver metallic effect with coat weight of 18g/m².

### Example 33d

Application by hand-coater (50µm wet film thickness) of the ink of example 33 on untreated wood (thickness 0.5cm) provides an opaque silver metallic effect.

### Example 34

A mixture of 35 parts of the varnish of example 32a) and 15 parts of XYMARA^{®} Gold Pearl G03 is gently stirred. 50 parts of the concentrate of example 32b) are added to this dispersion to form a homogeneous ink.
Application by hand-coater (50µm wet film thickness) of the ink of example 34 on AMCOR cardboard provides an opaque gold metallic effect with coat weight of 16g/m². Measurements performed with Datacolor Multi FX 10 apparatus on this print are represented in the table below.

| **Angles** | **L*** | **a*** | **b*** |
|---|---|---|---|
| **25°/170°** | **112.47** | **-0.38** | **14.46** |
| **25°/140°** | **115.22** | **3.06** | **35.61** |
| **45°/150°** | **110.56** | **4.61** | **38.93** |
| **45°/120°** | **98.61** | **7.31** | **42.08** |
| **75°/120°** | **88.49** | **8.43** | **39.37** |
| **75°/90°** | **81.55** | **8.37** | **36.37** |
| **45°/110°** | **69.69** | **4.91** | **27.38** |
| **45°/90°** | **39.34** | **2.47** | **11.81** |
| **45°/60°** | **23.85** | **1.79** | **6.76** |
| **45°/25°** | **19.32** | **2.04** | **5.76** |

### Example 35

A mixture of 35 parts of the varnish of example 32a) and 15 parts of XYMARA^{®} Bronze Pearl B04 is gently stirred. 50 parts of the concentrate of example 32b) are added to this dispersion and the thus obtained homogeneous ink has a viscosity of 62 sec (DIN Cup N°4). Application by hand-coater (50µm wet film thickness) of the ink of example 35 on AMCOR cardboard provides an opaque bonze metallic effect.

### Example 36

A mixture of 35 parts of the varnish of example 32a) and 15 parts of XYMARA^{®} Dual Pearl D31 is gently stirred. 50 parts of the concentrate of example 32b) are added to this dispersion and the thus obtained homogeneous ink has a viscosity of 40 sec (DIN Cup N°4). Application by hand-coater (50µm wet film thickness) of the ink of example 36 on AMCOR cardboard provides an opaque green metallic effect.

### Example 37

A mixture of 35 parts of the varnish of example 32a) and 15 parts of XYMARA^{®} Dual Pearl D21 is gently stirred. 50 parts of the concentrate of example 32b) are added to this dispersion and the thus obtained homogeneous ink has a viscosity of 39 sec (DIN Cup N°4). Application by hand-coater (50µm wet film thickness) of the ink of example 37 on AMCOR cardboard provides an opaque blue metallic effect with coat weight of 14.5g/m².

### Example 38

A mixture of 35 parts of the varnish of example 32a) and 15 parts of XYMARA^{®} Dual Pearl D19 is gently stirred. 50 parts of the concentrate of example 32b) are added to this dispersion and the thus obtained homogeneous ink has a viscosity of 60 sec (DIN Cup N°4). Application by hand-coater (50µm wet film thickness) of the ink of example 38 on AMCOR cardboard provides an opaque violet metallic effect.

### Example 39

a) One kilogram of aqueous clear varnish is prepared by mild stirring at 3000rpm for 30min at room temperature of a formulation containing 800g ARCOLOR binder (ARCOLOR AG), 8g TEGO Antifoam (DEGUSSA) and 192g water deionised. The thus obtained aqueous clear varnish has a viscosity of 14 sec (DIN Cup N°4).
b) An aqueous ink is prepared by dispersing in a Skandex^{®} for 2 hours in a 400 ml glass bottle 30 parts of compound A described in example 4 of PCT/EP2009/052127 where the dispersant is PLURONIC 123 (2%) and 230g of glass beads of 2mm diameter into 50 parts of the clear varnish prepared according to example 39a). Centrifugation and removal of the glass beads afford a concentrate of compound A and the thus obtained homogeneous ink has a viscosity of 20 sec (DIN Cup N°4).

### Example 40

A mixture of 35 parts of the varnish of example 39a) and 15 parts of XYMARA^{®} Silver Pearl S23 is gently stirred. 50 parts of the concentrate of example 39b) are added to this dispersion and the thus obtained homogeneous ink has a viscosity of 41 sec (DIN Cup N°4). Application by hand-coater (40µm wet film thickness) on laminate paper provides an opaque silver metallic effect with coat weight of 16g/m².
Measurements performed with Datacolor Multi FX 10 apparatus on the print of example 40 are represented in the table below.

| **Angles** | **L*** | **a*** | **b*** |
|---|---|---|---|
| **25°/170°** | **117.67** | **-0.11** | **-3.04** |
| **25°/140°** | **137.56** | **-0.85** | **-8.79** |
| **45°/150°** | **131.19** | **-0.75** | **-8.83** |
| **45°/120°** | **124.32** | **-0.60** | **-8.34** |
| **75°/120°** | **109.27** | **-0.95** | **-7.75** |
| **75°/90°** | **107.11** | **-0.80** | **-7.85** |
| **45°/110°** | **99.69** | **-0.46** | **-5.86** |
| **45°/900** | **60.05** | **-0.21** | **-2.56** |
| **45°/600** | **35.39** | **-0.19** | **-2.01** |
| **45°/25°** | **32.14** | **-0.72** | **-3.31** |

### Example 41

A mixture of 35 parts of the varnish of example 39a) and 15 parts of XYMARA^{®} Gold Pearl G03 is gently stirred. 50 parts of the concentrate of example 39b) are added to this dispersion and the thus obtained homogeneous ink has a viscosity of 33 sec (DIN Cup N°4). Application by hand-coater (40µm wet film thickness) on laminate paper provides an opaque gold metallic effect with coat weight of 16g/m².

### Example 42

A mixture of 35 parts of the varnish of example 39a) and 15 parts of XYMARA^{®} Bronze Pearl B04 is gently stirred. 50 parts of the concentrate of example 39b) are added to this dispersion and the thus obtained homogeneous ink has a viscosity of 50 sec (DIN Cup N°4). Application by hand-coater (40µm wet film thickness) on laminate paper provides an opaque bronze metallic effect with coat weight of 17.5g/m².

### Example 43

A mixture of 35 parts of the varnish of example 39a) and 15 parts of XYMARA^{®} Dual Pearl D31 is gently stirred. 50 parts of the concentrate of example 39b) are added to this dispersion and the thus obtained homogeneous ink has a viscosity of 20 sec (DIN Cup N°4). Application by hand-coater (40µm wet film thickness) on laminate paper provides an opaque green metallic effect with coat weight of 17g/m².

### Example 44

A mixture of 35 parts of the varnish of example 39a) and 15 parts of XYMARA^{®} Dual Pearl D21 is gently stirred. 50 parts of the concentrate of example 39b) are added to this dispersion and the thus obtained homogeneous ink has a viscosity of 23 sec (DIN Cup N°4). Application by hand-coater (40µm wet film thickness) on laminate paper provides an opaque blue metallic effect with coat weight of 19g/m².

### Example 45

A mixture of 35 parts of the varnish of example 39a) and 15 parts of XYMARA^{®} Dual Pearl D19 is gently stirred. 50 parts of the concentrate of example 39b) are added to this dispersion and the thus obtained homogeneous ink has a viscosity of 31 sec (DIN Cup N°4). Application by hand-coater (40µm wet film thickness) on laminate paper provides an opaque violet metallic effect with coat weight of 19g/m².

## Claims

1. A pigment mixture comprising two different components A and B, wherein component A is a graphite in the form of platelets (graphite nanoplatelets), which have an average particle size of below 50 µm (microns) and a thickness below 90 nm, and component B is an organic, or inorganic pigment.

2. The pigment mixture according to claim 1, wherein greater than 95% of the graphite nanoplatelets have a thickness below 50 nm.

3. The pigment mixture according to claim 2, wherein greater than 95% of the graphite nanoplatelets have a thickness below 20 nm.

4. The pigment mixture according to claim 1, wherein greater than 90% of the graphite nanoplatelets have a thickness of from about 3 nm to about 20 nm and a length and width of from about 1 to about 30 µm (microns).

5. The pigment mixture according to any of claims 1 to 4, wherein the component B is an effect pigment, especially a pearlescent pigment, a metal effect pigment, an interference pigment and/or a luster pigment.

6. The pigment mixture according to claim 5, wherein the component B is a platelet-like particle and has a (multilayer) structure comprising at least:
| | | | |
|---|---|---|---|
| TRASUB | TiO₂ | | |
| TRASUB | TiO₂ | Fe₂O₃ | |
| TRASUB | TiO₂ | Fe₃O₄ | |
| TRASUB | titanium suboxide(s) | | |
| TRASUB | TiO₂ | TiN | |
| TRASUB | TiO₂ | SiO₂ | |
| TRASUB | TiO₂ | titanium suboxide(s) | |
| TRASUB | TiO₂ | TiON | TiN |
| TRASUB | TiO₂ | SiO₂ | TiO₂ |
| TRASUB | TiO₂ | SiO₂ | silicon suboxide(s) |
| TRASUB | TiO₂ | SiO₂ | Fe₂O₃ |
| TRASUB | TiO₂ | SiO₂ | TiO₂/Fe₂O₃ |
| TRASUB | TiO₂ | SiO₂ | (Sn,Sb)O₂ |
| TRASUB | SnO₂ | TiO₂ | |
| TRASUB | SnO₂ | TiO₂ | Fe₂O₃ |
| TRASUB | (Sn,Sb)O₂ | SiO₂ | TiO₂ |
| TRASUB | Fe₂O₃ | SiO₂ | (Sn,Sb)O₂ |
| TRASUB | TiO₂/Fe₂O₃ | SiO₂ | TiO₂/Fe₂O₃ |
| TRASUB | Cr₂O₃ | SiO₂ | TiO₂ |
| TRASUB | Fe₂O₃ | | |
| TRASUB | Fe₂O₃ | SiO₂ | TiO₂ |
| TRASUB | titanium suboxide(s) | SiO₂ | titanium suboxide(s) |
| TRASUB | TiO₂ | SiO₂ | TiO₂ + SiO₂ + TiO₂ |
| TRASUB | TiO₂ + SiO₂ + TiO₂ | SiO₂ | TiO₂ + SiO₂ + TiO₂ |
| TRASUB | TiO₂ | Al₂O₃ | TiO₂ |
| TRASUB | Fe₂TiO₅ | SiO₂ | TiO₂ |
| TRASUB | TiO₂ | SiO₂ | Fe₂TiO₅/TiO₂ |
| TRASUB | TiO₂ | SiO₂ | MoS₂ |
| TRASUB | TiO₂ | SiO₂ | Cr₂O₃ |
| TRASUB | TiO₂ | SiO₂ | TiO₂ + SiO₂ + TiO₂ + Prussian Blue |
| TRASUB | TiO₂ | STL | |
wherein TRASUB is a semitransparent, or transparent substrate having a low index of refraction selected from the group consisting of natural, or synthetic mica, another layered silicate, glass, Al₂O₃, SiO₂, especially SiO₂, SiO₂/SiOₓ/SiO₂(0.03 ≤ x ≤ 0.95), SiO_{1.40-2.0}/SiO_{0.70-0.99}/SiO_{1.40-20}, or Si/SiO_{z} with 0.70 ≤ z ≤ 2.0, and
STL Is a semi-transparent layer selected from the group consisting of a semi-transparent metal layer of Cu, Ag, Cr, or Sn, or a semi-transparent silicon suboxide(s), titanium suboxide(s) or carbon layer.

7. The pigment mixture according to any of claims 1 to 4, wherein component B is an organic pigment in plateletlike form, such as a plateletlike quinacridone, phthalocyanine, fluororubine, dioxazine, red perylene or diketopyrrolopyrrole.

8. The pigment mixture according to claim 5, wherein the effect pigment is a platelet-like material, such as, for example, natural or synthetic micas, glass platelets, Al₂O₃ platelets, or SiO₂ platelets, coated with colored or colorless metal oxides, such as, for example, titanium dioxide, zirconium dioxide, and/or iron oxides.

9. The pigment mixture according to claim 8, wherein the effect pigment is natural or synthetic mica, glass platelets, Al₂O₃ platelets, or SiO₂ platelets, coated with titanium dioxide or zirconium dioxide.

10. The pigment mixture according to claim 8, wherein the effect pigment is natural or synthetic mica, glass platelets, Al₂O₃ platelets, or SiO₂ platelets, coated with Fe₂O₃ or Fe₃O₄.

11. The pigment mixture according to any of claims 1 to 10, where the weight ratio of component A to component B is in a ratio of from about 1:10 to about 10:1.

12. A coating, varnish, a plastic, a paint, a printing ink, a masterbatch, a ceramic or glass, cosmetic or personal care product, comprising the pigment mixture according to any of claims 1 to 11.

13. A method of forming a pigment mixture according to claim 1, comprising the steps of:
mixing the two components A and B to form a pigment composition, or
spray-drying an aqueous suspension consisting of discrete particles of component A and B.

14. Use of the pigment mixtures according to any of claims 1 to 11 in paints, for dyeing textiles, for pigmenting coatings, printing inks, plastics, cosmetics, wood coating/printing, metal coating printing, glazes for ceramics and glass.

15. A method for pigmenting high molecular weight organic material by incorporating an effective pigmenting amount of a pigment mixture according to any of claims 1 to 11 into said high molecular weight organic material.

## Patentansprüche

1. Pigmentmischung, umfassend zwei verschiedene Komponenten A und B, wobei es sich bei Komponente A um einen Graphit in Plättchenform (Graphit-Nanoplättchen) mit einer mittleren Teilchengröße von weniger als 50 µm (Mikron) und einer Dicke von weniger als 90 nm handelt und es sich bei Komponente B um ein organisches oder anorganisches Pigment handelt.

2. Pigmentmischung nach Anspruch 1, wobei mehr als 95% der Graphit-Nanoplättchen eine Dicke von weniger als 50 nm aufweisen.

3. Pigmentmischung nach Anspruch 2, wobei mehr als 95% der Graphit-Nanoplättchen eine Dicke von weniger als 20 nm aufweisen.

4. Pigmentmischung nach Anspruch 1, wobei mehr als 90% der Graphit-Nanoplättchen eine Dicke von etwa 3 nm bis etwa 20 nm und eine Länge und Breite von etwa 1 bis etwa 30 µm (Mikron) aufweisen.

5. Pigmentmischung nach einem der Ansprüche 1 bis 4, wobei es sich bei Komponente B um ein Effektpigment, insbesondere ein Perlglanzpigment, ein Metalleffektpigment, ein Interferenzpigment und/oder ein Glanzpigment, handelt.

6. Pigmentmischung nach Anspruch 5, wobei die Komponente B ein plättchenartiges Teilchen ist und eine (mehrschichtige) Struktur aufweist, die mindestens Folgendes umfasst:
| | | | |
|---|---|---|---|
| TRASUB | TiO₂ | | |
| TRASUB | TiO₂ | Fe₂O₃ | |
| TRASUB | TiO₂ | Fe₃O₄ | |
| TRASUB | Titan-suboxid(e) | | |
| TRASUB | TiO₂ | TiN | |
| TRASUB | TiO₂ | SiO₂ | |
| TRASUB | TiO₂ | Titan-suboxid(e) | |
| TRASUB | TiO₂ | TiON | TiN |
| TRASUB | TiO₂ | SiO₂ | TiO₂ |
| TRASUB | TiO₂ | SiO₂ | Silicium-suboxid(e) |
| TRASUB | TiO₂ | SiO₂ | Fe₂O₃ |
| TRASUB | TiO₂ | SiO₂ | TiO₂/Fe₂O₃ |
| TRASUB | TiO₂ | SiO₂ | (Sn, Sb)O₂ |
| TRASUB | SnO₂ | TiO₂ | |
| TRASUB | SnO₂ | TiO₂ | Fe₂O₃ |
| TRASUB | (Sn, Sb)O₂ | SiO₂ | TiO₂ |
| TRASUB | Fe₂O₃ | SiO₂ | (Sn, Sb)O₂ |
| TRASUB | TiO₂/Fe₂O₃ | SiO₂ | TiO₂/Fe₂O₃ |
| TRASUB | Cr₂O₃ | SiO₂ | TiO₂ |
| TRASUB | Fe₂O₃ | | |
| TRASUB | Fe₂O₃ | SiO₂ | TiO₂ |
| TRASUB | Titan-suboxid(e) | SiO₂ | Titan-suboxid(e) |
| TRASUB | TiO₂ | SiO₂ | TiO₂+SiO₂+TiO₂ |
| TRASUB | TiO₂+SiO₂+TiO₂ | SiO₂ | TiO₂+SiO₂+TiO₂ |
| TRASUB | TiO₂ | Al₂O₃ | TiO₂ |
| TRASUB | Fe₂TiO₅ | SiO₂ | TiO₂ |
| TRASUB | TiO₂ | SiO₂ | Fe₂TiO₅/TiO₂ |
| TRASUB | TiO₂ | SiO₂ | MoS₂ |
| TRASUB | TiO₂ | SiO₂ | Cr₂O₃ |
| TRASUB | TiO₂ | SiO₂ | TiO₂+SiO₂+TiO₂ + Preußischblau |
| TRASUB | TiO₂ | STL | |
Wobei TRASUB für ein halbtransparentes oder transparentes Substrat mit einem niedrigen Brechungsindex aus der Gruppe bestehend aus natürlichem oder synthetischem Glimmer, einem anderen Schichtsilicat, Glas, Al₂O₃, SiO₂, insbesondere SiO₂, SiO₂/SiOₓ/SiO₂ (0,03 ≤ x ≤ 0,95), SiO_{1,40-2,0}/SiO_{0,70-0,99}/Si_{1,40-2,0} oder Si/SiO_{z} mit 0, 70 ≤ z ≤ 2,0 steht und
STL für eine halbtransparente Schicht aus der Gruppe bestehend aus einer halbtransparenten Metallschicht aus Cu, Ag, Cr oder Sn oder einer halbtransparenten Schicht aus Siliciumsuboxid(en), Titansuboxid(en) oder Kohlenstoff steht.

7. Pigmentmischung nach einem der Ansprüche 1 bis 4, wobei es sich bei Komponente B um ein organisches Pigment in Plättchenform, wie plättchenförmiges Chinacridon, Phthalocyanin, Fluororubin, Dioxazin, rotes Perylen oder Diketopyrrolopyrrol handelt.

8. Pigmentmischung nach Anspruch 5, wobei es sich bei den Effektpigment um ein plättchenförmiges Material, wie beispielsweise natürliche oder synthetische Glimmer, Glasplättchen, Al₂O₃-Plättchen oder SiO₂-Plättchen, die mit farbigen oder farblosen Metalloxiden, wie beispielsweise Titandioxid, Zirconiumdioxid und/oder Eisenoxide, beschichtet sind, handelt.

9. Pigmentmischung nach Anspruch 8, wobei es sich bei dem Effektpigment um natürlichen oder syntheti-schen Glimmer, Glasplättchen, Al₂O₃-Plättchen oder SiO₂-Plättchen, die mit Titandioxid oder Zirconium-dioxid beschichtet sind, handelt.

10. Pigmentmischung nach Anspruch 8, wobei es sich bei dem Effektpigment um natürlichen oder syntheti-schen Glimmer, Glasplättchen, Al₂O₃-Plättchen oder SiO₂-Plättchen, die mit Fe₂O₃ oder Fe₃O₄ beschichtet sind, handelt.

11. Pigmentmischung nach einem der Ansprüche 1 bis 10, wobei das Gewichtsverhältnis von Komponente A zu Komponente B in einem Verhältnis von etwa 1:10 bis etwa 10:1 liegt.

12. Beschichtung, Lack, Kunststoff, Anstrichmittel, Druckfarbe, Masterbatch, Keramik oder Glas, Kosmetik- oder Körperpflegeprodukt, umfassend die Pigmentmischung nach einem der Ansprüche 1 bis 11.

13. Verfahren zur Bildung einer Pigmentmischung nach Anspruch 1, bei dem man:
die beiden Komponenten A und B zur Bildung einer Pigmentzusammensetzung mischt oder
eine wässrige Suspension, die aus diskreten Teilchen von Komponente A und B besteht, sprühtrocknet.

14. Verwendung der Pigmentmischungen nach einem der Ansprüche 1 bis 11 in Anstrichmitteln, zum Färben von Textilien, zum Pigmentieren von Beschichtungen, Druckfarben, Kunststoffen, Kosmetika, zum Beschichten/Bedrucken von Holz, zum Beschichten/Bedrucken von Metall und für Glasuren für Keramiken und Glas.

15. Verfahren zum Pigmentieren von hochmolekularem organischem Material durch Einarbeitung einer pigmentierungswirksamen Menge einer Pigmentmischung nach einem der Ansprüche 1 bis 11 in das hochmolekulare organische Material.

## Revendications

1. Mélange de pigments comprenant deux composants différents A et B, le composant A étant un graphite sous la forme de plaquettes (nanoplaquettes de graphite) qui ont une taille moyenne de particules inférieure à 50 µm (microns) et une épaisseur inférieure à 90 nm, et le composant B étant un pigment organique ou inorganique.

2. Mélange de pigments selon la revendication 1, dans lequel plus de 95 % des nanoplaquettes de graphite ont une épaisseur inférieure à 50 nm.

3. Mélange de pigments selon la revendication 2, dans lequel plus de 95 % des nanoplaquettes de graphite ont une épaisseur inférieure à 20 nm.

4. Mélange de pigments selon la revendication 1, dans lequel plus de 90 % des nanoplaquettes de graphite ont une épaisseur d'environ 3 nm à environ 20 nm et une longueur et une largeur d'environ 1 à environ 30 µm (microns).

5. Mélange de pigments selon l'une quelconque des revendications 1 à 4, dans lequel le composant B est un pigment à effet, en particulier un pigment nacré, un pigment à effet métallique, un pigment interférentiel et/ou un pigment brillant.

6. Mélange de pigments selon la revendication 5, dans lequel le composant B est une particule en forme de plaquette et a une structure (multicouche) comprenant au moins :
| | | | |
|---|---|---|---|
| TRASUB | TiO₂ | | |
| TRASUB | TiO₂ | Fe₂O₃ | |
| TRASUB | TiO₂ | Fe₃O₄ | |
| TRASUB | sous-oxyde(s) de titane | | |
| TRASUB | TiO₂ | TiN | |
| TRASUB | TiO₂ | SiO₂ | |
| TRASUB | TiO₂ | sous-oxyde(s) de titane | |
| TRASUB | TiO₂ | TiON | TiN |
| TRASUB | TiO₂ | SiO₂ | TiO₂ |
| TRASUB | TiO₂ | SiO₂ | sous-oxyde(s) de titane |
| TRASUB | TiO₂ | SiO₂ | Fe₂O₃ |
| TRASUB | TiO₂ | SiO₂ | TiO₂/Fe₂O₃ |
| TRASUB | TiO₂ | SiO₂ | (Sn, Sb)O₂ |
| TRASUB | SnO₂ | TiO₂ | |
| TRASUB | SnO₂ | TiO₂ | Fe₂O₃ |
| TRASUB | (Sn, Sb)O₂ | SiO₂ | TiO₂ |
| TRASUB | Fe₂O₃ | SiO₂ | (Sn, Sb)O₂ |
| TRASUB | TiO₂/Fe₂O₃ | SiO₂ | TiO₂/Fe₂O₃ |
| TRASUB | Cr₂O₃ | SiO₂ | TiO₂ |
| TRASUB | Fe₂O₃ | | |
| TRASUB | Fe₂O₃ | SiO₂ | TiO₂ |
| TRASUB | sous-oxyde(s) de titane | SiO₂ | sous-oxyde(s) de titane |
| TRASUB | TiO₂ | SiO₂ | TiO₂+SiO₂+TiO₂ |
| TRASUB | TiO₂+SiO₂+TiO₂ | SiO₂ | TiO₂+SiO₂+TiO₂ |
| TRASUB | TiO₂ | Al₂O₃ | TiO₂ |
| TRASUB | Fe₂TiO₅ | SiO₂ | TiO₂ |
| TRASUB | TiO₂ | SiO₂ | Fe₂TiO₅/TiO₂ |
| TRASUB | TiO₂ | SiO₂ | MoS₂ |
| TRASUB | TiO₂ | SiO₂ | Cr₂O₃ |
| TRASUB | TiO₂ | SiO₂ | TiO₂+SiO₂+TiO₂+ bleu de Prusse |
| TRASUB | TiO₂ | STL | |
dans lequel TRASUB est un substrat semi-transparent ou transparent ayant un faible indice de réfraction choisi dans le groupe constitué par un mica naturel ou synthétique, un autre silicate stratifié, du verre, Al₂O₃, SiO_{z}, en particulier SiO₂, SiO₂/SiOₓ/SiO₂ (0,03 ≤ x ≤ 0,95), SiO_{1,40-2,0}/SiO_{0,70-0,99}/Si_{1,40-2,0}, et Si/SiO_{z} avec 0,70 ≤ z ≤ 2,0, et
STL est une couche semi-transparente choisie dans le groupe constitué par une couche semi-transparente métallique de Cu, Ag, Cr ou Sn, et une couche semitransparente de sous-oxyde(s) de silicium, de sousoxyde(s) de titane ou de carbone.

7. Mélange de pigments selon l'une quelconque des revendications 1 à 4, dans lequel le composant B est un pigment organique en forme de plaquettes, tel que de la quinacridone, de la phtalocyanine, de la fluororubine, de la dioxazine, du rouge de pérylène ou du dicétopyrrolopyrrole en forme de plaquettes.

8. Mélange de pigments selon la revendication 5, dans lequel le pigment à effet est un matériau en forme de plaquettes, par exemple du mica naturel ou synthétique, des plaquettes de verre, des plaquettes d' Al₂O₃, ou des plaquettes de SiO₂, recouverts d'oxydes métalliques colorés ou incolores, par exemple du dioxyde de titane, du dioxyde de zirconium et/ou des oxydes de fer.

9. Mélange de pigments selon la revendication 8, dans lequel le pigment à effet est du mica naturel ou synthétique, des plaquettes de verre, des plaquettes d' Al₂O₃, ou des plaquettes de SiO₂, recouverts de dioxyde de titane ou de dioxyde de zirconium.

10. Mélange de pigments selon la revendication 8, dans lequel le pigment à effet est du mica naturel ou synthétique, des plaquettes de verre, des plaquettes d' Al₂O₃, ou des plaquettes de SiO₂, recouverts de Fe₂O₃ ou de Fe₃O₄.

11. Mélange de pigments selon l'une quelconque des revendications 1 à 10, dans lequel le rapport pondéral entre le composant A et le composant B est un rapport d'environ 1:10 à environ 10:1.

12. Revêtement, vernis, plastique, peinture, encre d'impression, mélange-maître, céramique ou verre, produit cosmétique ou de soins personnels, comprenant le mélange de pigments selon l'une quelconque des revendications 1 à 11.

13. Procédé de formation d'un mélange de pigments selon la revendication 1, comprenant les étapes consistant à :
mélanger les deux composants A et B pour former une composition de pigments, ou
sécher par atomisation une suspension aqueuse constituée de particules discrètes de composant A et B.

14. Utilisation des mélanges de pigments selon l'une quelconque des revendications 1 à 11 dans des peintures, pour teindre des textiles, pour pigmenter des revêtements, des encres d'impression, des plastiques, des cosmétiques, le revêtement/l'impression du bois, le revêtement/l'impression du métal, des glaçures pour les céramiques et le verre.

15. Procédé de pigmentation d'un matériau organique à haut poids moléculaire par incorporation d'une quantité de pigmentation efficace d'un mélange de pigments selon l'une quelconque des revendications 1 à 11 dans ledit matériau organique à haut poids moléculaire.
